# EUROPÄISCHE PATENTSCHRIFT

(11) **EP 4 504 396 B1**
(45) Veröffentlichungstag und Bekanntmachung des Hinweises auf die Patenterteilung: **02.07.2025**
(21) Anmeldenummer: 23721594.2
(22) Anmeldetag: 11.04.2023
(51) Int. Cl.: B01J 19/12

(54) **LED-LAMPENMODUL UND VORRICHTUNG ZUR DURCHFÜHRUNG EINER PHOTOCHEMISCHEN REAKTION**
LED LAMP MODULE AND DEVICE FOR CARRYING OUT A PHOTOCHEMICAL REACTION
MODULE DE LAMPE À DEL ET DISPOSITIF POUR EFFECTUER UNE RÉACTION PHOTOCHIMIQUE

(30) Priorität: 07.04.2022 WO PCT/EP2022/059279
(43) Veröffentlichungstag der Anmeldung: 12.02.2025
(73) Patentinhaber: Peschl Ultraviolet GmbH, 55130 Mainz (DE)
(72) Erfinder: PESCHL, Alexander, 55130 Mainz (DE)
(74) Vertreter: mepat Patentanwälte
(86) Internationale Anmeldenummer: PCT/EP2023/059385
(87) Internationale Veröffentlichungsnummer: WO 2023/194621

(56) Entgegenhaltungen:
- WO-A1-2016/026576
- WO-A1-2020/228980
- DE-A1- 102014 012 218
- DE-B4- 102014 012 219

## Beschreibung

Die vorliegende Erfindung bezieht sich auf ein LED-Lampenmodul, das zum Einsatz in einer Vorrichtung zur Durchführung einer photochemischen Reaktion im industriellen Maßstab mit einer hohen LED-Anzahl bzw. LED-Dichte ausgebildet ist, und auf eine Vorrichtung zur Durchführung einer photochemischen Reaktion, die zumindest ein solches LED-Lampenmodul aufweist.

Bislang werden zur Durchführung photochemischer Reaktionen im industriellen Maßstab, in dem Reaktionsräume auch mehrere Meter durchmessen können, häufig immer noch Niederdruck- oder Mitteldruckstrahler eingesetzt, da diese Abmessungen in entsprechender Größenordnung aufweisen können. Allerdings ist deren Betrieb mit hohem Stromverbrauch verbunden und mit zunehmendem Alter sind regelmäßige Überprüfungen in relativ kurzen Zeitabständen erforderlich, da die Strahlungsintensität deutlich abnimmt und das Strahlungsspektrum sich verschieben kann.

Daher ist man bestrebt, auch im photochemischen Bereich LED-Lampen einzusetzen, die im Vergleich mit den herkömmlichen Niederdruck- oder Mitteldruckstrahlern einen geringen Stromverbrauch, eine hohe Lebensdauer und eine hohe Schaltfestigkeit bei spontan vollem Lichtstrom haben. Als photochemische Reaktion gelten vorliegend sämtliche durch Photonen induzierte Prozesse, z. B. photochemische oder photokatalysierte Synthesen, sogenannte AOPs ("advanced oxidation processes") und UV-Desinfektion von Wasser und Oberflächen.

Sollen LEDs in Photoreaktoren für chemische Synthesen, z. B. Photochlorierung oder Photobromierung als Tauchlampe zum Einsatz kommen, so muss das eingesetzte Lampenmodul nicht nur den erhöhten Anforderungen hinsichtlich der umgebenden Reaktionsbedingungen genügen, die sehr deutlich von Umgebungsdruck und Raumtemperatur abweichen können, sondern auch an die Größe des Reaktionsraums angepasste Abmessungen aufweisen. Der Einsatz von LEDs als Lampenmodul im industriellen Maßstab wird dabei durch die Anzahl und Dichte der LEDs begrenzt, die aufgrund ihrer Betriebsweise mit geringer Spannung und der für eine hohe Strahlungsleistung erforderlichen hohen Stromstärke möglich ist. Werden die LEDs für eine hohe Lichtausbeute bzw. Strahlungsintensität mit hohen Strömen betrieben, ist zur Vermeidung von lokal hohen Temperaturen eine effektive Wärmeableitung von den LEDs erforderlich, um deren Lebensdauer zu erhalten. Zur effektiven Wärmeabfuhr werden die LEDs an einem meist metallischen Trägerkörper angeordnet, der Strukturen zur Wärmeableitung wie beispielsweise Kühlkanäle aufweisen kann, durch die ein Kühlfluid geleitet wird. Auch eine direkte Kühlung von LEDs mittels elektrisch nichtleitender Kühlflüssigkeit ist bekannt. Aber auch bei effektiver Wärmeabfuhr ist der Betrieb der LEDs am Leistungsmaximum zur Durchführung photochemischer Reaktionen für die Lebensdauer der LEDs abträglich.

Aus DE 10 2014 012 219 B4 ist zur Anpassung der Abmessungen für den Einsatz im industriellen Maßstab ein LED-Lampenmodul zur Durchführung photochemischer Reaktionen mit einer deutlich erhöhten Anzahl von LEDs bekannt. Dabei weist der Trägerkörper für die LEDs einen Kühlpfad auf, der über ein Kopfteil des Lampenmoduls mit einem Kühlkreislauf verbunden ist. Zur Verbesserung der Stromversorgung ist der Trägerkörper ferner mit zumindest einer längsaxialen oder achsparallelen Kammer ausgebildet, durch die sich eine Leitung zur Stromversorgung/Steuerung von dem Kopfteil bis zu einem Kontaktelement der LEDs erstreckt. Die Stromversorgungs- und Steuerungsvorrichtung, die beispielsweise Vorschalt- bzw. Leistungselektronik, Treiber und Netzteile umfassen kann, ist dabei eine externe Stromversorgungs- und Steuerungsvorrichtung, die außerhalb des Photoreaktors angeordnet ist und über eine elektrische Anschlussvorrichtung des Kopfteils angeschlossen wird. Alternativ kann die Stromversorgungs- und Steuerungsvorrichtung im Kopfteil untergebracht sein, sodass die temperaturempfindliche Leistungselektronik bzw. die LED-Treiber durch Abschnitte des für die LEDs vorgesehenen Kühlkreislaufs gekühlt werden können.

Zum Einsatz als Tauchlampe in einer Vorrichtung zur Durchführung photochemischer Reaktionen wird ein LED-Lampenmodul üblicherweise in einem transparenten, meist einseitig geschlossenen Hüllrohr angeordnet. Das Kopfteil, über das die elektrische Versorgung des LED-Lampenmoduls erfolgt, kann das offene Ende des Hüllrohrs verschließen. Der Trägerkörper kann einen sechs- oder achteckigen Querschnitt haben und damit sechs oder acht Seitenflächen bereitstellen, an denen LEDs befestigt werden können. Dazu sind die LEDs üblicherweise in einer vorbestimmten Anzahl auf Platinen angeordnet, die entsprechende Leiterbahnen zum Anschluss der LEDs aufweisen. Die an einer Seitenfläche des Trägerkörpers angeordneten Platinen bilden jeweils einen LED-Strang, der mit einem Treiber betrieben wird. Beispielsweise liegt bei einer Strangspannung von 300 V und einer Vorwärtsspannung der LEDs von 4 V die maximale Anzahl der LEDs pro Strang bei 75 Stück, die mit einem Treiber betrieben werden können. Mit einer derzeit maximalen Baulänge der Platinen von ca. 50 cm, die jeweils mit 18 oder 15 LEDs bestückt sind, bilden 4 oder 5 Platinen hintereinander geschaltet einen Strang, sodass sich eine maximale Länge des Lampenmoduls von 200 bis 250 cm ergibt. Ist an jeder Seitenfläche des sechs- oder achteckigen Trägerkörpers jeweils ein LED-Strang mit maximaler LED-Anzahl vorgesehen, weist die LED-Lampe 450 oder 600 LEDs auf.

Die mit der Anzahl von 75 LED pro Strang auf eine Länge von 200 bis 250 cm erreichbare Dichte von 0,375 bis 0,3 LED/cm kann je nach Prozess und gewünschter Homogenität deutlich zu wenig sein. Eine höhere Dichte, d. h. eine höhere Anzahl LEDs pro cm Leuchtlänge ist aber aufgrund der Limitierung durch 300 V Vorschaltgerätespannung pro Strang nur durch Verkürzung des LED-Lampenmoduls zu erreichen, was der gewünschten Verlängerung des Lampenmoduls zur Anpassung an die Abmessungen der Reaktionsräume im industriellen Maßstab widerspricht. Hinzu kommt, dass für jedes Vorschaltgerät bzw. Treiber zwei Anschlusskontakte erforderlich sind, die z. B. über Stecker in das Kopfteil der Lampe eingebracht werden. Ist an jeder Seitenfläche eines sechs- oder achteckigen Trägerkörpers jeweils ein LED-Strang vorgesehen, sind entsprechend sechs oder acht Vorschaltgeräte/Treiber erforderlich, die dann 12 oder 16 Anschlusskontakte benötigen. Bei gegebenem Durchmesser der Lampe bzw. des Kopfteils ist der zur Verfügung stehende Platz für Anschlüsse begrenzt. Damit ist auch die Anzahl der Vorschaltgeräte bzw. Treiber limitiert, die an einer LED-Lampe zum Betrieb jeweils eines LED-Strangs angeschlossen werden können.

Ein Upscaling von LED-Lampen, die eine deutlich höhere Anzahl an LEDs aufweisen sollen, um eine zur Durchführung einer photochemischen Reaktion erforderliche hohe LED-Dichte bereitzustellen, insbesondere auch bei einer Lampenlänge von über 200 bis 250 cm, ist daher nicht ohne weiteres möglich, da für die sehr hohe LED-Anzahl eine entsprechend große Anzahl an Vorschaltgeräten/Treibern mit jeweils zwei Anschlusskontakten erforderlich wäre und der Platz für Anschlüsse am Kopfteil der Lampe begrenzt ist. WO2016/026576 offenbart einen photochemischen Reaktor mit einem Lampenmodul. Das Lampenmodul weist eine Vielzahl von LEDs in LED-Gruppen auf.

Ausgehend von diesem Stand der Technik liegt der vorliegenden Erfindung die Aufgabe zugrunde, ein skalierbares LED-Lampenmodul ausgebildet für photochemische Reaktionen im industriellen Maßstab mit einer verbesserten LED-Dichte bereitzustellen.

Diese Aufgabe wird durch ein LED-Lampenmodul mit den Merkmalen des Anspruchs 1 gelöst.

Die weitere Aufgabe, eine Vorrichtung mit einem verbesserten LED-Lampenmodul zur Durchführung einer photochemischen Reaktion bereitzustellen, wird durch die Vorrichtung mit den Merkmalen des unabhängigen Anspruchs 15 gelöst.

Weiterbildungen und bevorzugte Ausführungsformen sind in den Unteransprüchen ausgeführt.

Gemäß einer ersten Ausführungsform weist das erfindungsgemäße LED-Lampenmodul, das zur Anordnung in einer Vorrichtung zur Durchführung einer photochemischen Reaktion ausgebildet ist, eine Vielzahl LEDs und einen formgebenden Trägerkörper auf. Die LEDs sind an dem Trägerkörper befestigt, der mit den LEDs in einem Innenraum des LED-Lampenmoduls angeordnet ist, der von einem transparenten Wandelement und von einem Gehäuseelement begrenzt wird. Das transparente Wandelement ist korrespondierend zu den LEDs angeordnet, und durch das Gehäuseelement erstreckt sich zumindest eine elektrische Versorgungsleitung zum elektrischen Anschluss der LEDs. Dabei ist die Vielzahl LEDs in LED-Gruppen unterteilt, die eine vorbestimmte Anzahl LEDs aufweisen, wobei die LEDs jeder LED-Gruppe in Reihe geschaltet sind, und jede LED-Gruppe jeweils einer Treibervorrichtung als Konstantstromquelle zum Betrieb der LEDs der jeweiligen LED-Gruppe zugeordnet und mit dieser verbunden ist. Erfindungsgemäß ist jede Treibervorrichtung benachbart zu der ihr zugeordneten LED-Gruppe an dem Trägerkörper im Innenraum des LED-Lampenmoduls angeordnet, wobei jede Treibervorrichtung mit der jeweils zugeordneten LED-Gruppe in Reihe geschaltet ist und einen LED- Stromzweig bildet. Die durch die Treibervorrichtungen mit den LED-Gruppen gebildeten LED- Stromzweige sind dabei parallel geschaltet, wobei die parallel geschalteten LED-Stromzweige über die Versorgungsleitung mit einer Konstantspannungsquelle verbunden sind. Die Konstantspannung der Konstantspannungsquelle wird von der Treibervorrichtung jeder LED-Gruppe in Konstantstrom umgewandelt.

Durch die erfindungsgemäß gemischte Schaltung zur Versorgung der LEDs, die sowohl Elemente von Reihenschaltungen als auch Parallelschaltungen enthält, kann das LED-Lampenmodul einfach durch Vervielfachung der parallelen LED-Stromzweige skaliert und so die Anzahl der LEDs deutlich erhöht werden, ohne dass die Anzahl der dafür benötigten Versorgungsleitungen mit den elektrischen Anschlüsse am Gehäuseelement zunimmt, da die Spannung der parallelen LED-Stromzweige konstant ist.

Mit dem Begriff der Versorgungsleitung werden hierin Hin- und Rückleiter des LED-Stromkreises mit den parallel geschalteten LED-Stromzweigen zusammengefasst. Ein LED-Lampenmodul kann dabei mehr als einen LED-Stromkreis mit jeweils einer Versorgungsleitung wie vorstehend aufweisen. An dem Gehäuseelement ist für jede Versorgungsleitung eine Anschlussvorrichtung vorgesehen, die entsprechend zwei Anschlusskontakte für Hin- und Rückleiter der Versorgungsleitung aufweist. Die Versorgungsleitung mit Hin- und Rückleiter kann in einem Kabel über einen Anschlussstecker angeschlossen werden, der die zwei Anschlusskontakte aufweist. Es kann aber auch vorgesehen sein, dass für Hin- und Rückleitung einer Versorgungsleitung separate Kabel vorgesehen sind, die jeweils einen Anschlussstecker mit einem der Anschlusskontakte aufweisen.

Vorteilhaft kann ein erfindungsgemäßes LED-Lampenmodul aufgrund dieser gemischten Schaltung mit den integrierten Treibervorrichtungen nicht nur in einer gewünschten Länge ausgeführt werden, auch von mehr als 200 cm, sondern auch bezüglich LED-Dichte, Leistungsdichte und LED-Vorwärtsspannung variabel an die Erfordernisse der jeweiligen photochemischen Reaktion angepasst werden. Denn die zur Durchführung einer photochemischen Reaktion erforderliche Leistungsdichte kann stark variieren. Beispielsweise sind zur Durchführung einer Photochlorierungsreaktion LED-Lampenmodule mit hoher LED-Dichte und geringer Leistung geeignet, während die Durchführung einer Photoredoxreaktion LED-Lampenmodule erfordert, die eine hohe LED-Dichte mit hoher Leistung aufweisen. Bevorzugt kann ein erfindungsgemäßes LED-Lampenmodul aufgrund dieser gemischten Schaltung mit den integrierten Treibervorrichtungen mit einer LED-Dichte von zumindest 125 LEDs/m und/oder einer Leistungsdichte von zumindest 35 W/m und/oder einer LED-Vorwärtsspannung von zumindest 3 V pro LED in der gewünschten Länge, auch von mehr als 200 cm ausgeführt werden.

Weitere Ausführungsformen des erfindungsgemäßen LED-Lampenmoduls sehen vor, dass die vorbestimmte Anzahl LEDs aller LED-Gruppen gleich ist und höchstens 25 beträgt. Mit einer beispielhaften Vorwärtsspannung jeder LED von 4 V liegt dann die Konstantspannung der Konstantspannungsquelle bei höchstens 100 V. Bevorzugt ist es möglich, dass die vorbestimmte Anzahl LEDs höchstens 20 beträgt, sodass die Konstantspannung der Konstantspannungsquelle mit der beispielhaften Vorwärtsspannung jeder LED von 4 V bei höchstens 80 V liegt. Insbesondere beträgt die vorbestimmte Anzahl LEDs höchstens 15, sodass die Konstantspannung der Konstantspannungsquelle mit der beispielhaften Vorwärtsspannung jeder LED von 4 V höchstens 60 V beträgt. Aufgrund der parallel geschalteten LED-Gruppen mit zugeordneter Treibervorrichtung kann die Konstantspannung im Vergleich zum Stand der Technik deutlich reduziert werden. Ausführungsformen mit einer vorteilhaft geringen Spannung von höchstens 60 V DC können bevorzugt sein, da dann ATEX-Steckverbinder mit geeigneter Dimensionierung zum Anschluss der Versorgungsleitung am Gehäuseelement eingesetzt werden können, sodass das LED-Lampenmodul in explosionsgefährdeten Bereichen eingesetzt werden kann, wenn z. B. Edukte oder Produkte der photochemischen Reaktion mit Luft eine explosionsfähige Atmosphäre bilden können.

Gemäß einer weiteren Ausführungsform des erfindungsgemäßen LED-Lampenmoduls können die LED-Stromzweige aus den LED-Gruppen mit den ihnen zugeordneten Treibervorrichtungen als integrierte Schaltbausteine an Platinen, die an dem Trägerkörper befestigt sind, ausgebildet sein, wobei an jeder Platine eine oder mehrere der LED-Gruppen mit der jeweiligen Treibervorrichtung angeordnet ist/sind. Auf diese Weise ist mit den Platinen ein modulares System geschaffen, mit dem eine LED-Lampe flexibel gestaltet und erweitert werden kann. Es bestehen keine Beschränkungen hinsichtlich der Länge des Lampenmoduls oder der Anzahl der LED-Stromzweige bzw. der Platinen.

Ferner kann ein erfindungsgemäßes LED-Lampenmodul dahingehend weitergebildet sein, dass jeder LED-Gruppe zumindest ein Steuer- und/oder Regelelement und zumindest eine Messvorrichtung zugeordnet ist, die ausgewählt ist aus einer Gruppe, die eine Strommessvorrichtung, eine Spannungsmessvorrichtung, eine Temperaturmessvorrichtung und eine Photonenmessvorrichtung enthalten kann. Alternativ zu der zumindest einen Messvorrichtung oder zusätzlich dazu kann jeder LED-Gruppe ferner zumindest ein Schalterelement zugeordnet sein. Das Steuer- und/oder Regelelement ist dabei mit der zumindest einen Messvorrichtung kommunikativ verbunden und dazu ausgebildet, in Abhängigkeit eines von der zumindest einen Messvorrichtung erfassten Messwertes das Schalterelement anzusteuern, um die LEDs der zugeordneten LED-Gruppe in Abhängigkeit der Messwerte an- oder abzuschalten und/oder zu dimmen. Selbstverständlich ist dazu das Steuer- und/oder Regelelement auch mit dem zumindest einen Schalterelement kommunikativ verbunden. Ferner kann das Steuer- und/oder Regelelement dazu ausgebildet sein, die Treibervorrichtung der zugeordneten LED-Gruppe zur Regelung des Konstantstroms anzusteuern, um die LEDs der zugeordneten LED-Gruppe mit einer vorbestimmten Leistung zu betreiben.

Durch die Integration einer Strom- und Spannungsmessung für jede LED-Gruppe auf der Platine kann ein korrekter Betrieb sichergestellt werden. Im Fehlerfall, z. B. Ausfall einer LED in einer LED-Gruppe, der durch die integrierte Strom- und Spannungsmessung erkannt wird, kann die zugeordnete LED-Gruppe abgeschaltet werden und so vermieden werden, dass die übrigen LEDs der Gruppe überlastet werden. Durch den Ausfall einer LED entsteht ein Hotspot, der zum Ausfall weiterer LEDs und damit zu weiteren Hotspots führen kann, die in einer explosionsfähigen Atmosphäre, in der sich das LED-Lampenmodul befinden kann, durch die Abschaltung zuverlässig vermieden werden kann, sodass der Explosionsschutz verbessert wird. Ein Hotspot kann auch mit der integrierten Temperaturmessung festgestellt werden, die aber vor allem dazu dient, dass die LEDs, deren optischer Output nicht nur strom- sondern auch temperaturabhängig ist, die gewünschte Strahlungsleistung liefern. Daher kann mit der Temperaturmessung ein Betrieb der LEDs innerhalb eines vorbestimmten Temperaturfensters sichergestellt werden, sodass verhindert wird, dass die LEDs zu warm werden. Zur Justierung des optischen Outputs der LED-Gruppen kann auch die optionale Photonenmessung genutzt werden.

Nach einer weiteren Ausführungsform können die Messvorrichtung und/oder das Schalterelement wie die Treibervorrichtung als integrierte Schaltbausteine auf der Platine der zugeordneten LED-Gruppe angeordnet sein.

Ebenso kann das Steuer- und/oder Regelelement nach einer weiteren Ausführungsform eines erfindungsgemäßen LED-Lampenmoduls als integrierter Schaltbaustein auf der Platine der zugeordneten LED-Gruppe ausgebildet sein. Alternativ kann jedes einer LED-Gruppe zugeordnete Steuer- und/oder Regelelement außerhalb des Innenraums angeordnet sein. In beiden Fällen sind die Steuer- und/oder Regelelemente aller LED-Gruppen miteinander und/oder mit einer übergeordneten Steuer- und/oder Regeleinheit kommunikativ verbunden und dazu ausgebildet, die vorbestimmte Strahlungsleistung der LEDs jeder LED-Gruppe aufeinander abzustimmen, um eine homogene Gesamtstrahlungsleistung zu erreichen. Alternativ können einzelne LED-Gruppen an- und abgeschaltet werden, um nur Teile des LED-Lampenmoduls zur Strahlungsemission zu verwenden. Ferner können die miteinander und/oder mit einer übergeordneten Steuer- und/oder Regeleinheit verbundenen Steuer- und/oder Regeleinheiten dazu ausgebildet sein, jeder LED-Gruppe eine eindeutige ID zuzuweisen, und/oder Betriebsdaten und/oder Betriebszeit jeder LED-Gruppe zu erfassen.

Eine weitere Ausführungsform des erfindungsgemäßen LED-Lampenmoduls kann vorsehen, dass das LED-Lampenmodul zumindest eine Versorgungsschiene zur Aufnahme der zumindest einen Versorgungsleitung innerhalb des Innenraums aufweist.

Nach einer bevorzugten Ausführungsform des erfindungsgemäßen LED-Lampenmoduls ist der Trägerkörper ein Tragkäfig mit einer Gitterstruktur, der Tragstrukturen, an denen die LED-Gruppen befestigt sind, und zumindest eine Stützstruktur zur positionierten Anordnung der an dem Tragkäfig befestigten LED-Gruppen innerhalb des Innenraums aufweist.

Gemäß einer vorteilhaften Ausführungsform kann die zumindest eine Versorgungsschiene durch zumindest eine Schienenstruktur des Tragkäfigs bereitgestellt werden, sodass durch die Versorgungsleitung(en) die LEDs nicht verschatten.

Ferner kann ein erfindungsgemäßes LED-Lampenmodul nach einer weiteren Ausführungsform einen Kühlkreislauf mit einer elektrisch nichtleitenden Flüssigkeit aufweisen, wobei das Gehäuseelement einen Zulaufanschluss und einen Ablaufanschluss zur Verbindung des Innenraums mit dem Kühlkreislauf aufweist, sodass der Innenraum vollständig mit der elektrisch nichtleitenden Flüssigkeit gefüllt ist. Damit können die Treibervorrichtungen und die LEDs sowie ggf. die weiteren Schaltbausteine durch die elektrisch nichtleitende Flüssigkeit gemeinsam gekühlt werden. Diese Kühlung ist besonders effektiv, wenn der Trägerkörper als Tragkäfig ausgeführt ist.

Nach einer speziellen Ausführungsform ist das erfindungsgemäße LED-Lampenmodul als Tauchlampe mit einer Längsachse ausgebildet, wobei das transparenten Wandelement ein transparentes, einseitig geschlossenes Hüllrohr ist, das koaxial um den Trägerkörper mit den LEDs angeordnet ist. Das Gehäuseelement weist ein Kopfteil auf, das an einem offenen Ende des Hüllrohrs angeordnet ist.

In einer Weiterbildung davon weist der als Tragkäfig ausgebildete Trägerkörper an zumindest einem längsaxialen Ende die zumindest eine Stützstruktur zur positionierten Anordnung innerhalb des Innenraums auf, wobei die Stützstruktur zur zentrierten Anordnung an dem Kopfteil und/oder dem geschlossenen Ende des Hüllrohrs ausgebildet ist. Alternativ oder zusätzlich weist das Kopfteil Durchtrittsöffnungen auf, wobei sich der Zulaufanschluss und der Ablaufanschluss jeweils durch eine der Durchtrittsöffnungen erstrecken oder damit verbunden sind. Dabei ist der Zulaufanschluss bevorzugt innenraumseitig mit einer Rohrstruktur verbunden, die in dem als Tragkäfig ausgebildeten Trägerkörper angeordnet ist und sich längs durch den Tragkäfig als Kühlmittelzulaufabschnitt erstreckt, der eine Zulauföffnung an einem kopfteilfernen Ende des Innenraums bereitstellt. An einem kopfteilnahen Ende des Innenraums wird eine Ablauföffnung durch den Ablaufanschluss, der sich durch das Kopfteil erstreckt, oder durch die mit dem Ablaufanschluss verbundene Durchtrittsöffnung bereitgestellt.

Alternativ zu einer Tauchlampe kann ein erfindungsgemäßes LED-Lampenmodul nach einer weiteren Ausführungsform als Flächenstrahler ausgebildet sein, wobei das transparente Wandelement eine transparente ebene Fensterscheibe ist, und das Gehäuseelement ein Gehäuse ist, das eine Basis und Seitenwände aufweist. Die Fensterscheibe schließt eine von Seitenwände begrenzte offene Fläche, wobei die an dem Trägerkörper angeordneten LEDs in einer zu der Fensterscheibe parallelen Ebene zwischen der Fensterscheibe und der Basis angeordnet sind.

Nach einer Weiterbildung der erfindungsgemäßen Vorrichtung ist der als Tragkäfig ausgebildete Trägerkörper mit der zumindest einen Stützstruktur auf der Basis des Gehäuses angeordnet, wobei die Tragstrukturen, an denen die LEDs befestigt sind, auf einer von der zumindest einen Stützstruktur abgewandten Seite des Tragkäfigs ausgebildet sind. Dabei bestimmt eine Abmessung des Tragkäfigs zwischen den Tragstrukturen und der zumindest einen Stützstruktur einen Abstand der LEDs von der Fensterscheibe. Alternativ oder zusätzlich weist das Gehäuse auf gegenüberliegenden Seiten des Innenraums zumindest eine Zulauföffnung, die mit dem Zulaufanschluss verbunden ist, und zumindest eine Ablauföffnung auf, die mit dem Ablaufanschluss verbunden ist.

Eine ebenfalls erfindungsgemäße Vorrichtung zur Durchführung einer photochemischen Reaktion weist gemäß einer ersten Ausführungsform einen Photoreaktor auf, an oder in dem zumindest ein Lampenmodul angeordnet ist, das Strahlung mit einer für die photochemische Reaktion geeigneten Wellenlänge emittiert, wobei das Lampenmodul ein erfindungsgemäßes LED-Lampenmodul ist.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine schematische Darstellung einer Vorrichtung zur Durchführung einer photochemischen Reaktion mit einem LED-Lampenmodul aus dem Stand der Technik,
- **Fig. 2**: eine schematische Darstellung einer erfindungsgemäßen Vorrichtung zur Durchführung einer photochemischen Reaktion mit einem erfindungsgemäßen LED-Lampenmodul,
- **Fig. 3**: eine schematische Darstellung einer LED-Gruppe eines erfindungsgemäßen LED-Lampenmoduls,
- **Fig. 4**: eine teilgeschnittene Seitenansicht eines erfindungsgemäßen LED-Lampenmoduls in einer Ausführung als Tauchlampe,
- **Fig. 5**: eine Querschnittsansicht des erfindungsgemäßen LED-Lampenmoduls aus Fig. 4 entlang Schnittlinie XX,
- **Fig. 6**: eine Draufsicht auf ein erfindungsgemäßes LED-Lampenmodul in einer Ausführung als Flächenstrahler,
- **Fig. 7**: eine Längsschnittansicht des erfindungsgemäßen LED-Lampenmoduls aus Fig. 6 entlang Schnittlinie YY,
- **Fig. 8**: eine schematische Darstellung einer erfindungsgemäßen Vorrichtung zur Durchführung einer photochemischen Reaktion mit einem erfindungsgemäßen LED-Lampenmodul in der Ausführung als Tauchlampe,
- **Fig. 9**: eine schematische Darstellung einer erfindungsgemäßen Vorrichtung zur Durchführung einer photochemischen Reaktion mit einem erfindungsgemäßen LED-Lampenmodul in der Ausführung als Flächenstrahler.

Die vorliegende Erfindung bezieht sich auf ein LED-Lampenmodul, das zum Einsatz in einer Vorrichtung zur Durchführung einer photochemischen Reaktion mit einer dafür angepassten LED-Dichte, Leistungsdichte und LED-Vorwärtsspannung in einer gewünschten Länge, die auch mehr als 200 cm betragen kann, ausgebildet ist.

Das LED-Lampenmodul kann eine LED-Dichte von zumindest 125 LEDs/m und/oder eine Leistungsdichte von zumindest 35 W/m und/oder eine Vorwärtsspannung von zumindest 3 V pro LED aufweisen.

Ferner bezieht sich die vorliegende Erfindung auch auf eine Vorrichtung zur Durchführung einer photochemischen Reaktion, die ein solches erfindungsgemäßes LED-Lampenmodul aufweist.

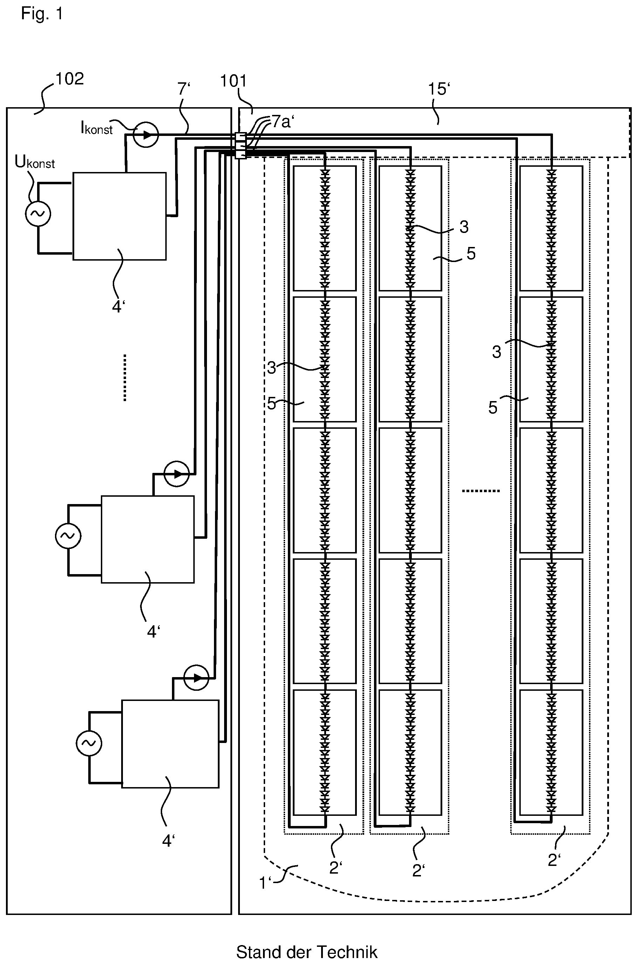

**Im Stand der Technik** wird zur Durchführung einer photochemischen Reaktion ein LED-Lampenmodul 1' eingesetzt, das wie in **Fig. 1** schematisch dargestellt als Tauchlampe in einem Photoreaktor 101 eingesetzt werden kann, um ein darin vorgelegtes Medium einer elektromagnetischen Strahlung auszusetzen, die die gewünschte photochemische Reaktion hervorruft. Für einen Umsatz im industriellen Maßstab ist es erforderlich, dass Photoreaktoren Abmessungen von mehreren Metern aufweisen, die entsprechend dimensionierte Lampen erfordern, um eine gleichmäßige Bestrahlung des Reaktorvolumens zu erreichen. Im Gegensatz zu herkömmlichen Niederdruck- oder Mitteldruckstrahlern, die mit entsprechenden Abmessungen verfügbar sind, ist die Länge der LED-Lampenmodule 1' des Stands der Technik bei einer für eine Mindestleistung vorgegebenen LED-Dichte begrenzt.

Ein LED-Strang 2' kann maximal 75 in Reihe geschaltete LEDs 3 aufweisen, die von einem Treiber 4', der einen Konstantstrom Iₖₒₙₛₜ bereitstellt, mit einer Strangspannung von maximal 300 V betrieben werden. Werden diese 75 LEDs auf vier oder fünf Platinen 5 mit einer maximalen Platinenlänge von 50 cm verteilt, kann das LED-Lampenmodul 1' bzw. der LED-Strang 2' zwar eine Leuchtenlänge von 200 oder 250 cm erreichen. Dies ist allerdings mit einer LED-Dichte von 37,5 LEDs/m bzw. 30 LEDs/m verbunden, was je nach Prozess und gewünschter Homogenität deutlich zu wenig sein kann. Eine höhere LED-Dichte wäre zwar mit kürzeren LED-Lampenmodulen 1' möglich, aber nachteilig mit einer ungleichmäßigen Ausleuchtung eines entsprechend großvolumigen Photoreaktors 101 verbunden.

Eine weitere Limitierung des LED-Lampenmoduls 1' aus dem **Stand der Technik** besteht darin, dass für jeden LED-Strang 2' jeweils ein Treiber 4' vorzusehen ist. Ein LED-Lampenmodul 1' mit sechs oder acht LED-Strängen 2' benötigt entsprechend sechs oder acht Treiber 4', die üblicherweise von dem Photoreaktor 101 beabstandet in einem separaten Schaltschrank 102 untergebracht sind, um mit einer Spannungsquelle Uₖₒₙₛₜ verbunden zu werden. Und da ferner das LED-Lampenmodul 1' an dem Kopfteil 15' für jeden LED-Strang 2' zum Anschluss an den zugeordneten Treiber 4' über eine Versorgungsleitung 7' eine Anschlussvorrichtung 7a' mit jeweils zwei Anschlusskontakten für Hin- und Rückleiter der Versorgungsleitung 7' aufweisen muss, ist die Anzahl der LED-Stränge 2' durch den am LED-Lampenmodul 1' für Anschlusskontakte verfügbaren Platz begrenzt.

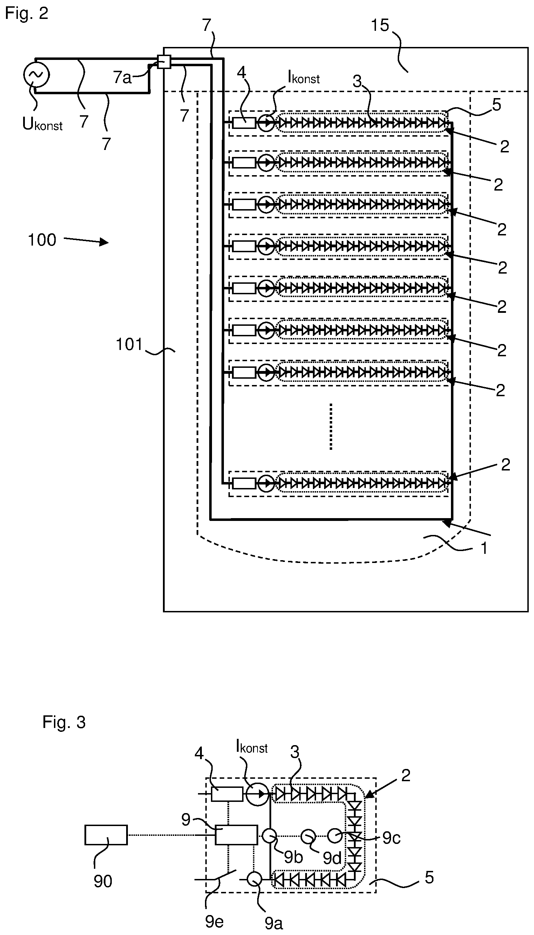

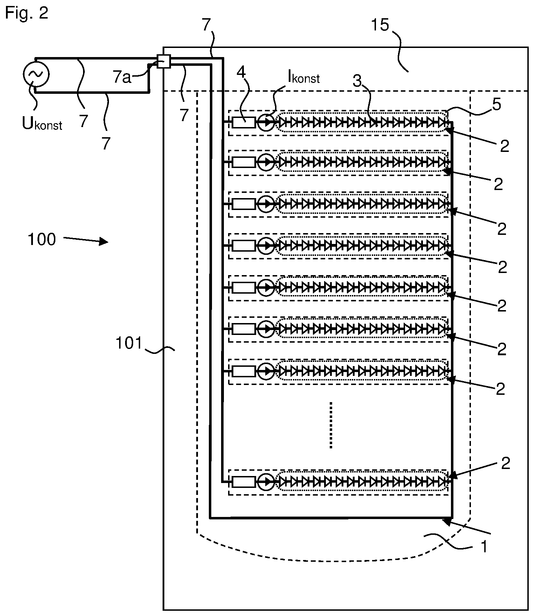

Das erfindungsgemäß ausgeführte LED-Lampenmodul 1, für das unterschiedliche Beispiele in **Fig. 2 bis 7** gezeigt sind, überkommt die Limitierungen des Standes der Technik: Das LED-Lampenmodul 1 weist zum Einsatz in Vorrichtungen 100 zur Durchführung photochemischer Reaktionen eine von der Lampenlänge unabhängige LED-Dichte von zumindest 125 LEDs/m und/oder eine Leistungsdichte von zumindest 35 W/m und/oder eine Vorwärtsspannung von zumindest 3 V pro LED auf.

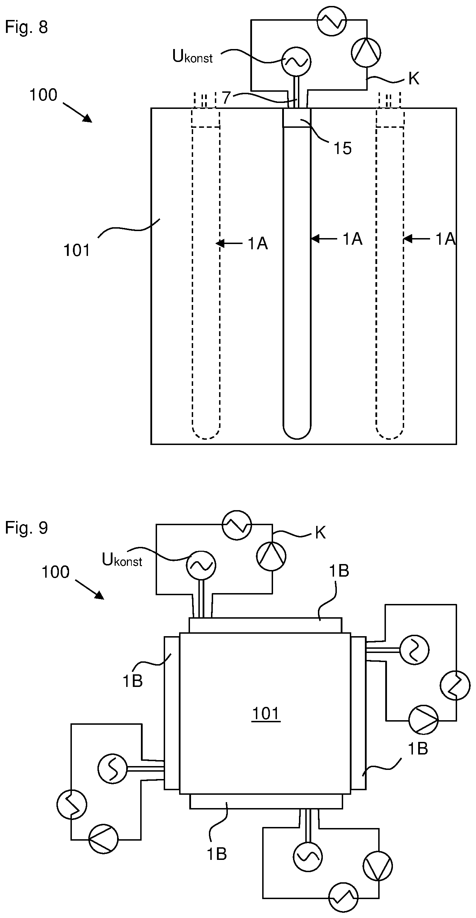

In **Fig. 2** ist der Einsatz eines LED-Lampenmoduls 1 in einer Vorrichtung 100 zur Durchführung einer photochemischen Reaktion schematisch dargestellt, **Fig. 8 und 9** zeigen weitere Beispiele von Vorrichtungen 100 zur Durchführung einer photochemischen Reaktion mit unterschiedlich ausgeführten LED-Lampenmodulen 1. In jedem LED-Lampenmodul 1 ist eine Vielzahl LEDs 3 an einem Trägerkörper 6 befestigt, dessen Form an die Geometrie des LED-Lampenmoduls 1 angepasst ist und der zumindest eine Oberfläche zur Befestigung der LEDs 3 bereitstellt. Durch die Orientierung der Befestigungsoberfläche wird eine Abstrahlrichtung der LEDs 3 vorgegeben, mit der die Abstrahlcharakteristik des LED-Lampenmoduls 1 definiert wird. Dabei kann die Abstrahlcharakteristik durch Einsatz von Reflexions-, Refraktions- und/oder Filterelementen modifiziert werden.

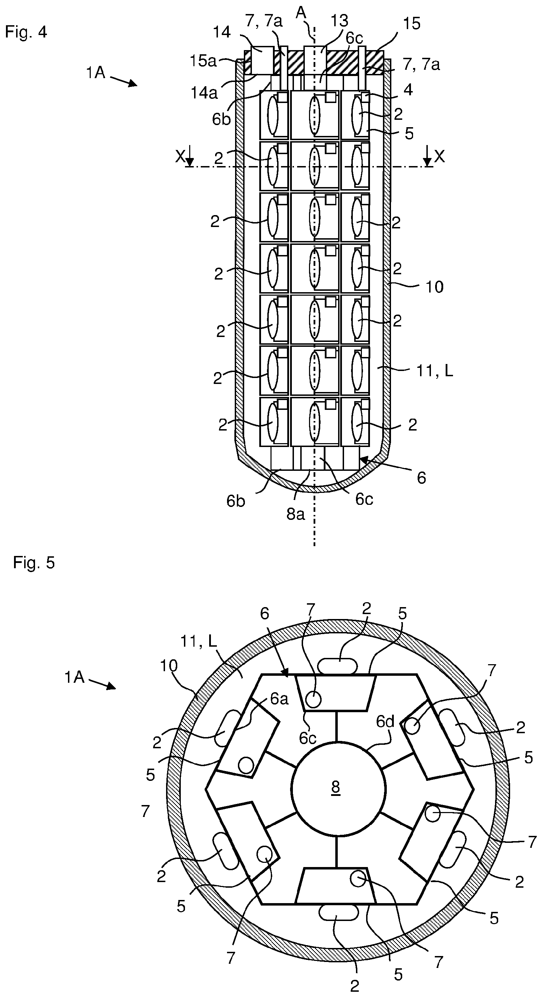

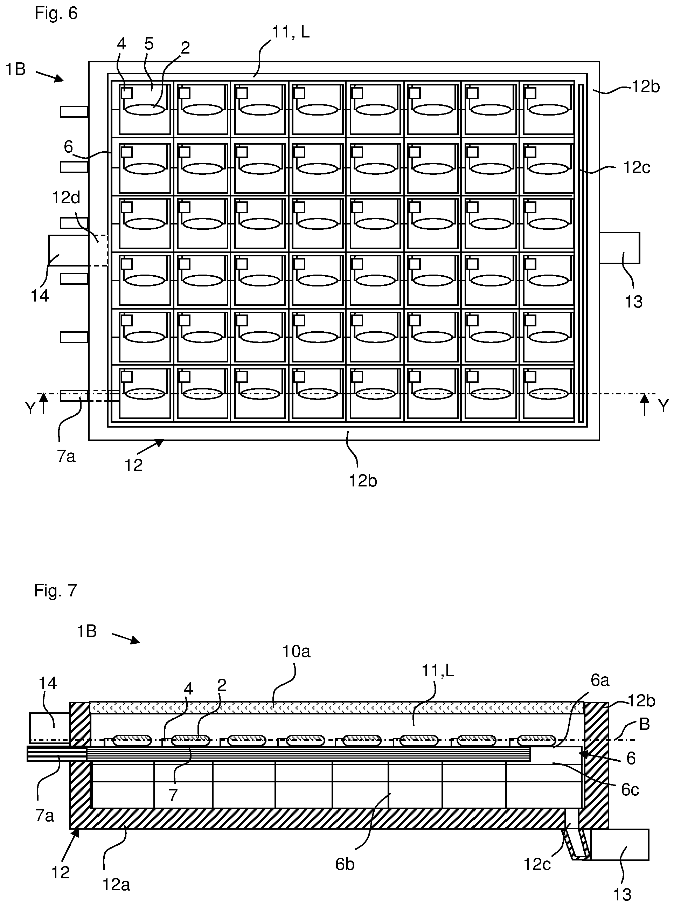

**Fig. 4 und 5** zeigt ein Beispiel für ein als Tauchlampe 1A ausgebildetes LED-Lampenmodul 1, wobei der Trägerkörper 6 die Form eines geometrischen Prismas mit der Längsachse A aufweist. Alternativ kann der Trägerköper einer Tauchlampe auch Zylinderform haben. Die LEDs 3 sind in LED-Gruppen 2 rundum an der Mantelfläche des prismatischen Trägerkörpers 6 befestigt, sodass die Abstrahlrichtungen der LEDs 3 der LED-Gruppen 2 der Tauchlampe 1A im Wesentlichen radial zur Längsachse A verlaufen. Ein weiteres, in **Fig. 6 und 7** dargestelltes Beispiel ist ein als Flächenstrahler 1B ausgebildetes LED-Lampenmodul 1, bei dem der Trägerkörper 6 die Form eines Quaders aufweist, der mit einer Seitenfläche eine ebene Befestigungsfläche zur Anordnung der LEDs 3 in einer Ebene B bereitstellt, sodass die Abstrahlrichtung der LEDs 3 des LED-Flächenstrahlers 1B im Wesentlichen orthogonal zur Ebene B verläuft. In den **Fig. 4 bis 7** ist jede LED-Gruppe 2 vereinfacht als Ellipse dargestellt, der Übersichtlichkeit wegen wurde darin auf eine Darstellung der einzelnen LEDs 3 wie in **Fig. 2 und 3** verzichtet.

Es versteht sich, dass ein erfindungsgemäßes LED-Lampenmodul 1 auch eine von den beiden Beispielen abweichende Form des Trägerkörpers aufweisen kann, um je nach Einsatzzweck eine modifizierte Lampengeometrie für eine andere Hauptabstrahlrichtungen zu erhalten. Ferner muss nicht die gesamte Mantelfläche eines prismatischen bzw. zylindrischen Trägerkörpers als Befestigungsflächen für die LEDs genutzt werden, wenn etwa für die Tauchlampe keine rotationssymmetrische Abstrahlcharakteristik erwünscht oder erforderlich ist. Eine Abwandlung des Flächenstrahlers hingegen kann vorsehen, dass mehr als einer Seitenfläche des quaderförmigen Trägerkörpers als Befestigungsfläche eingesetzt werden, um z. B. Abstrahlrichtungen in entgegengesetzte Richtungen orthogonal zur Ebene B oder Abstrahlrichtungen orthogonal und parallel zur Ebene B zu erhalten.

Jedes LED-Lampenmodul 1 weist ferner ein transparentes Wandelement 10, 10a und ein Gehäuseelement 12, 15 auf, die zusammen einen Innenraum 11 begrenzen, in dem der Trägerkörper 6 mit den daran angeordneten LEDs 3 angeordnet ist, sodass das transparente Wandelement 10, 10a korrespondierend zu den LEDs 3 angeordnet ist, um die von den LEDs 3 emittierte Strahlung austreten zu lassen. Das jeweilige Gehäuseelement 12, 15 weist eine oder mehrere Anschlussvorrichtung(en) 7a zur Verbindung mit (jeweils) einer Versorgungsleitung 7 zum elektrischen Anschluss der LEDs 3 auf.

Bei der Tauchlampe 1A in **Fig. 4 und 5** ist das transparenten Wandelement ein einseitig geschlossenes Hüllrohr 10, das koaxial um den Trägerkörper 6 mit den LEDs 3 angeordnet ist. Das Gehäuseelement ist ein Kopfteil 15, das an dem offenen Ende des Hüllrohrs 10 angeordnet ist. Alternativ zu dem dargestellten Beispiel des einseitig geschlossenen Hüllrohrs 10, bei dem der Bodenabschnitt, der das kopfteilferne Ende schließt, integral mit der Hüllwandung ausgebildet ist, kann das kopfteilferne Ende eines beidseitig offenen Hüllrohrs durch einen Stopfen verschlossen sein.

Der Flächenstrahler 1B aus **Fig. 6 und 7** weist eine ebene, flächige Fensterscheibe 10a als transparentes Wandelement auf, während das Gehäuseelement als Gehäuse 12 ausgebildet ist, das eine Basis 12a und Seitenwände 12b aufweist. Die Seitenwände 12b begrenzen eine von der Basis 12a beabstandete offene Fläche, die durch die Fensterscheibe 10a geschlossen wird. Der Trägerkörper 6 ist auf der Basis 12a angeordnet, sodass die Befestigungsfläche mit den LEDs 3 zu der Fensterscheibe 10 weist und die an dem Trägerkörper 6 angeordneten LEDs 3 in einer zu der Fensterscheibe 10a parallelen Ebene B zwischen der Fensterscheibe 10a und der Basis 12a angeordnet sind. Ist in einer Abwandlung vorgesehen, dass mehr als einer Seitenfläche des quaderförmigen Trägerkörpers als Befestigungsfläche mit LEDs ausgebildet ist, kann das Gehäuse entsprechend modifiziert werden, um weitere Fensterscheiben korrespondierend zu den Befestigungsflächen mit LEDs anzuordnen. Dazu können die Basis und/oder die Seitenwände mit entsprechenden Fensteröffnungen zur Anordnung der weiteren Fensterscheiben gegenüber den LEDs ausgebildet sein.

Zum elektrischen Anschluss sind die LEDs 3 in LED-Gruppen 2 unterteilt, die eine vorbestimmte Anzahl in Reihe geschalteter LEDs 3 aufweisen, wie in **Fig. 2** zu sehen ist. In **Fig. 4 bis 7** sind der Übersichtlichkeit wegen nicht die einzelnen LEDs 3, sondern nur die LED-Gruppen 2 dargestellt. Jede LED-Gruppe 2 ist mit jeweils einer Treibervorrichtung 4 verbunden, die einen Konstantstrom Iₖₒₙₛₜ zum Betrieb der LEDs 3 der jeweiligen LED-Gruppe 2 bereitstellt. Dabei sind die Treibervorrichtungen 4 zusammen mit der jeweils zugeordneten LED-Gruppe 2 an dem Trägerkörper 6 im Innenraum 11 des LED-Lampenmoduls 1 angeordnet. Wie in **Fig. 2** gut zu sehen ist, bildet jede LED-Gruppe 2 mit der zugeordneten in Reihe geschalteten Treibervorrichtung 4 einen LED-Stromzweig, wobei die LED-Stromzweige parallel geschaltet und über die Versorgungsleitung 7 mit einer externen Konstantspannungsquelle Uₖₒₙₛₜ verbunden sind.

Durch die Parallelschaltung der LED-Gruppen 2, die über eine einzige Versorgungsleitung 7 versorgt werden können, verringert sich die Anzahl der am Gehäuseelement 15 für eine vorbestimmte LED-Anzahl benötigten Anschlussvorrichtungen 7a deutlich, da nicht mehr wie im Stand der Technik pro LED-Strang eine Anschlussvorrichtung benötigt wird. Das heißt jedoch nicht, dass ein erfindungsgemäßes LED-Lampenmodul 1 auf eine Ausführung mit einer Anschlussvorrichtung 7a für einen LED-Stromkreis mit einer Vielzahl paralleler LED-Stromzweige beschränkt ist, die jeweils eine Treibervorrichtung 4 mit einer LED-Gruppe 2 aufweisen. Vielmehr kann ein LED-Lampenmodul 1, wie das die Beispiele in **Fig. 4 bis 7** zeigen, mehrere LED-Stromkreise mit einer Vielzahl parallel geschalteter LED-Stromzweige aufweisen, wobei für die Versorgungsleitung 7 jedes LED-Stromkreises eine Anschlussvorrichtung 7a am Gehäuseelement 12, 15 vorgesehen ist. Dabei weist jede Anschlussvorrichtung zwei Anschlusskontakte für einen Hin- und Rückleiter der Versorgungsleitung 7 auf. Die beiden Anschlusskontakte können gemeinsam in einem Anschlussstecker oder in zwei Anschlusssteckern ausgebildet sein. Ohne die Anzahl der Anschlussvorrichtungen 7a erhöhen zu müssen, lässt sich das erfindungsgemäße LED-Lampenmodul 1 durch Vervielfachung der parallel geschalteten LED-Gruppen 2 mit zugeordnetem Treiber 4 einfach skalieren, insbesondere verlängern, ohne die LED-Dichte verringern zu müssen. Dadurch kann auch bei längeren Lampenmodulen hohe LED-Dichte bzw. Leistung umgesetzt und damit eine größtmögliche Homogenität der Bestrahlung erreicht werden.

Und anders als im Stand der Technik, in dem die LEDs am Leistungsmaximum betrieben werden, um eine für die photochemischen Reaktionen ausreichend hohe Photonenleistung zu erhalten, können die LEDs 3 des erfindungsgemäßen Lampenmoduls 1 mit hohem Vorwärtsstrom eingesetzt und gedimmt betrieben werden, um eine lange LED Lebensdauer zu gewährleisten.

Die an eine Versorgungsleitung 7 angeschlossenen, parallel geschalteten LED-Gruppen 2 weisen eine gleichbleibende Anzahl LEDs 3 auf, beispielsweise 15 wie in **Fig. 2 und 3****.** Mit einer Vorwärtsspannung von 4 V liegt die Versorgungsspannung der Konstantspannungsquelle Uₖₒₙₛₜ dann bei lediglich 60 V DC, sodass vorteilhaft auch ATEX-Steckverbinder zum Anschluss am Gehäuseelement eingesetzt werden können, und das LED-Lampenmodul 1 in einem Bereich mit potentiell explosionsfähiger Atmosphäre eingesetzt werden kann. Grundsätzlich ist es durchaus möglich, dass die LED-Gruppen 2 eines LED-Lampenmoduls 1 auch mehr als 15 LEDs aufweisen, sodass die Versorgungsspannung der Konstantspannungsquelle Uₖₒₙₛₜ bei einer Vorwärtsspannung von 4 V entsprechend höher liegt, solange das Gehäuseelement ausreichend Platz zum Anschluss der Versorgungsleitung(en) 7 bietet. Daher ist der Einsatz von ATEX-Steckverbindern, die zum Einsatz bei höheren Spannungen größere Abmessungen aufweisen, begrenzt und abhängig von der Dimensionierung des Gehäuseelements.

In den dargestellten Beispielen ist jede LED-Gruppe 2 mit der zugeordneten Treibervorrichtung 4 als integrierte Schaltbausteine auf einer Platine 5 ausgebildet, die an dem Trägerkörper 6 befestigt ist. In alternativen nicht dargestellten Ausführungsformen können aber auch mehrere LED-Gruppen 2 mit den zugeordneten Treibervorrichtungen 4 auf einer Platine 5 gemeinsam angeordnet sein.

Auf den Platinen 5 können, wie in **Fig. 3** schematisch dargestellt, auch Steuer- und/oder Regelelemente 9, Messvorrichtungen 9a, 9b, 9c, 9d sowie Schalterelemente 9e als integrierte Schaltbausteine ausgebildet sein, die einer jeweiligen LED-Gruppe 2 zugeordnet sind. Als Messvorrichtungen 9a, 9b, 9c, 9d können eine Strommessvorrichtung 9a, eine Spannungsmessvorrichtung 9b, eine Temperaturmessvorrichtung 9c und eine Photonenmessvorrichtung 9d eingesetzt werden, die aber nicht zwingend wie in **Fig. 3** dargestellt kombiniert eingesetzt werden müssen. Es ist auch möglich, einer LED-Gruppe 2 nur eine Strommessvorrichtung 9a und/oder eine Spannungsmessvorrichtung 9b zuzuordnen. Eine Temperaturmessvorrichtung 9c und/oder eine Photonenmessvorrichtung 9d können auch mehreren LED-Gruppen 2 zugeordnet oder gruppenunabhängig im Innenraum 11 an dem Trägerkörper 6 angeordnet sein.

Das einer LED-Gruppe 2 zugeordnete Steuer- und/oder Regelelement 9 ist nicht nur mit der jeweiligen Treibervorrichtung 4, sondern auch mit der oder den jeweiligen Messvorrichtungen 9a, 9b, 9c, 9d und mit dem Schalterelement 9e kommunikativ verbunden, um in Abhängigkeit eines von der jeweiligen Messvorrichtung 9a, 9b, 9c, 9d erfassten Messwertes das Schalterelement 9e und/oder die Treibervorrichtung 4 anzusteuern. Durch Ansteuerung des Schalterelements 9e werden die LEDs 3 der zugeordneten LED-Gruppe 2 in Abhängigkeit der Messwerte an- oder abgeschaltet. Beispielsweise kann die LED-Gruppe 2 abgeschaltet werden, wenn durch die Temperaturmessvorrichtung 9c eine zu hohe Temperatur und/oder durch die Strom- und/oder Spannungsmessvorrichtungen 9a, 9b Ausfälle einer oder mehrerer LEDs 3 der LED-Gruppe 2 erkannt werden. Die Treibervorrichtung 4 wird zur Regelung des Konstantstroms Iₖₒₙₛₜ angesteuert, um die LEDs 3 der zugeordneten LED-Gruppe 2 mit einer vorbestimmten Strahlungsleistung zu betreiben, etwa wenn die Photonenmessvorrichtung 9d eine zu geringe Photonendichte erfasst oder ein durch die Strom- und/oder Spannungsmessung erkannter Ausfall einer oder mehrerer LEDs 3 kompensiert werden soll.

Anders als im Beispiel von **Fig. 3****,** in dem das Steuer- und/oder Regelelement 9 als integrierter Schaltbaustein auf der Platine 5 der zugeordneten LED-Gruppe 2 ausgebildet ist, kann in einer nicht dargestellten Variante ein Steuer- und/oder Regelelement 9, das einer LED-Gruppe 2 zugeordnet ist, außerhalb des Innenraums 11 angeordnet sein. In beiden Fällen können die Steuer- und/oder Regelelemente 9 der LED-Gruppen 2 mit einer übergeordneten Steuer- und/oder Regeleinheit 90, wie in **Fig. 3** angedeutet, kommunikativ verbunden sein, um die vorbestimmte Strahlungsleistung der LEDs 3 jeder LED-Gruppe 2 für homogene Gesamtstrahlungsleistung aufeinander abzustimmen. Alternativ können die Steuer- und/oder Regelelemente 9 der LED-Gruppen 2 ohne übergeordnete Steuer- und/oder Regeleinheit 90 kommunikativ verbunden sein, um die vorbestimmte Strahlungsleistung der LEDs 3 jeder LED-Gruppe 2 für homogene Gesamtstrahlungsleistung aufeinander abzustimmen.

Des Weiteren kann durch die Steuer- und/oder Regelelemente 9 oder die übergeordnete Steuer- und/oder Regeleinheit 90 jeder LED-Gruppe 2 eine ID, etwa ein Identifikationscode, zugewiesen werden, um z. B. die Platine 5 zu identifizieren, bei der ein Ausfall einer LED 3 erfasst wurde, der mit dem Auge nicht erkennbar ist. Damit ist eine Abschaltung der betroffenen LED-Gruppe 2 im Fehlerfall möglich, wodurch vermieden wird, dass die übrigen LEDs 3 der betroffenen LED-Gruppe 2 überlastet werden. Ferner können Betriebsdaten und/oder Betriebszeit jeder LED-Gruppe 2 erfasst werden, um die Wartung des LED-Lampenmoduls 1 zu vereinfachen.

Wie in **Fig. 4 bis 7** zu erkennen ist, ist der Trägerkörper 6 nicht massiv ausgeführt, sondern als Tragkäfig 6 mit einer Gitterstruktur entsprechend einem Skelettbau ausgebildet, der aus Stab- und/oder Flächenelementen zusammensetzt ist, die miteinander verbundene Öffnungen umrahmen. Der Tragkäfig 6 weist dabei Tragstrukturen 6a zur Befestigung der LED-Gruppen 2 bzw. der Platinen 5 und Stützstrukturen 6b zur positionierten Anordnung innerhalb des Innenraums 11 auf. Die Tragstrukturen 6a des Tragkäfigs 6 werden durch Stab- und/oder Flächenelemente gebildet, die bei der LED-Tauchlampe 1A mit dem prismatischen Tragkäfig 6 in **Fig. 4 und 5** in der Mantelfläche des Prismas liegen. Die Stützstrukturen 6b, die ebenfalls aus Stab- und/oder Flächenelementen gebildet sind, dienen einerseits zur Befestigung des Tragkäfig 6 an dem Kopfteil 15 und andererseits zur Abstützung an dem geschlossenen Ende des Hüllrohrs 10 und sorgen damit für eine zentrierte Anordnung innerhalb des Hüllrohrs 10.

Bei dem LED-Flächenstrahler 1B mit dem quaderförmigen Tragkäfig 6 in **Fig. 6 und 7** sind die Tragstrukturen 6a und die Stützstrukturen 6b ebenfalls aus Stab- und/oder Flächenelementen gebildet, wobei sich die Tragstrukturen 6a an der Seitenfläche des Quaders befinden, die zu der Fensterscheibe 10a weist, und die Stützstrukturen 6b an den Seitenflächen, die zur Basis 12a und den Seitenwänden 12b des Gehäuses 12 weisen. Durch die Abmessung des Tragkäfigs 6 zwischen den Tragstrukturen 6a und den auf der Basis 12a aufliegenden Stützstrukturen 6b wird der Abstand der LEDs 3 von der Fensterscheibe 10a bestimmt.

Außerdem weist der Tragkäfig 6 eine Schienenstruktur 6c, ebenfalls aus Stab- und/oder Flächenelementen, auf, die eine Bus- oder Versorgungsschiene 6c bilden, in der die Versorgungsleitung 7 durch den Innenraum 11 zur Anschlussvorrichtung 7a am Gehäuseelement 12, 15 geführt wird. Durch die so bereitgestellte Kabelführung "hinter" den LEDs 3 bzw. den Platinen 5 werden Verschattungen der LEDs 3 vermieden.

Ein Tragkäfig 6 als Trägerkörper 6 für die LEDs 3 ist insbesondere dann vorteilhaft, wenn das LED-Lampenmodul 1 einen Kühlkreislauf K (vgl. **Fig. 8, 9**) zur Zirkulation einer elektrisch nichtleitenden Flüssigkeit L aufweist, die den Innenraum 11 und auch die Öffnungen des Tragkäfigs 6 vollständig füllt. Zur Verbindung des Innenraums 11 mit dem Kühlkreislauf K weist das Gehäuseelement 12, 15 einen Zulaufanschluss 13 und einen Ablaufanschluss 14 auf, die in **Fig. 4 bis 7** dargestellt sind. Auf diese Weise werden die LEDs 3 bzw. die Platinen 5 von beiden Seiten mit der elektrisch nichtleitenden Flüssigkeit L umströmt, um die von den LEDs 3 und der Treibervorrichtung 4 (und ggf. von weiteren Schaltbausteine auf der Platine 5) erzeugte Wärme aufzunehmen, die mittels des Kühlkreislaufs K außerhalb des LED-Lampenmoduls 1 abgegeben wird. Dazu weist der Kühlkreislauf K üblicherweise nebst einer Förderpumpe einen Wärmetauscher auf.

In der in **Fig. 4 und 5** dargestellten Ausführung als LED-Tauchlampe 1A weist das Kopfteil 15 Durchtrittsöffnungen 15a auf, durch die sich der Zulaufanschluss 13 und der Ablaufanschluss 14 erstrecken. Der Zulaufanschluss 13 ist dabei innenraumseitig mit einer Rohrstruktur 6d verbunden, die sich längsaxial durch den Tragkäfig 6 als Kühlmittelzulaufabschnitt 8 erstreckt. Damit stellt die Rohrstruktur 6d an einem kopfteilfernen Ende des Innenraums 11 eine Zulauföffnung 8a bereitstellt, durch den die elektrisch nichtleitende Flüssigkeit L, die über den Zulaufanschluss 13 zugeführt wird, aus dem Kühlmittelzulaufabschnitt 8 in den Innenraum 11 strömt. Zur Ausleitung der elektrisch nichtleitenden Flüssigkeit L aus dem Innenraum 11 ist an dem kopfteilnahen Ende des Innenraums 11 eine Ablauföffnung 14a vorgesehen, die durch den exzentrisch angeordneten Ablaufanschluss 14 bereitgestellt wird.

Bei dem LED-Flächenstrahler 1 B aus **Fig. 6 und 7** weist das Gehäuse 12 auf gegenüberliegenden Seiten des Innenraums 11 eine mit dem Zulaufanschluss 13 verbundene Zulauföffnung 12c und eine mit dem Ablaufanschluss 12 verbundene Ablauföffnung 12d auf. Die Zulauföffnung 12c erstreckt sich durch die Basis 12a direkt benachbart zu einer Seitenwand und verbreitert sich zu einem Schlitz parallel zur Seitenwand der in den Innenraum 11 mündet. Die Ablauföffnung 12d erstreckt sich mittig durch die gegenüberliegende Seitenwand. Diese Gestaltung von Zu- und Ablauföffnungen 12c, 12d ist beispielhaft zu verstehen, hierbei sind diverse Abwandlungen in Form und Anzahl der Öffnungen oder durch Verteiler- oder Sammlerelementen zwischen Anschlüssen und Öffnungen denkbar.

Auch die in **Fig. 8 und 9** gezeigten Vorrichtungen 100 zur Durchführung einer photochemischen Reaktion sind als Beispiele zu verstehen. Erfindungsgemäße Vorrichtungen 100 können mit einer abweichenden Anzahl und/oder Anordnung der LED-Lampenmodule 1 ausgebildet sein, die an oder in einem Photoreaktor 101 angeordnet sind. Anders als dargestellt, kann eine Vorrichtung 100 auch kombiniert LED-Lampenmodule 1 an und in dem Photoreaktor 101 aufweisen, um das darin vorgelegte Medium von außen und innen zu bestrahlen. Der Photoreaktor 101 kann dabei als Batch-Reaktor oder Durchflussreaktor ausgebildet sein. **Fig. 8** verdeutlicht die Anordnung von LED-Tauchlampen 1A im Inneren eines Photoreaktors 101, wobei ein Reaktionsmedium die Tauchlampe 1A umgibt. Das heißt, dass die Tauchlampe 1A in ein im Photoreaktor 101 befindliches Reaktionsmedium eingetaucht sein kann, sodass die von der Tauchlampe 1A emittierte Strahlung direkt in das Reaktionsmedium gelangt. Alternativ kann innerhalb des Photoreaktors 101 ein Reaktorvolumen, das das Reaktionsmedium enthält, durch eine transparente Wandung von der Tauchlampe 1A getrennt sein, etwa um eine thermische Entkopplung oder den Explosionsschutz zu verbessern oder um das Reaktionsmedium in einem bestimmten Strömungsweg entlang der Tauchlampe zu führen. Zur Trennung der Tauchlampe 1A zur Verbesserung der thermischen Entkopplung bzw. des Explosionsschutzes kann ein zusätzliches Tauchrohr (nicht dargestellt) um das Hüllrohr eingesetzt werden, wobei der dazwischen liegende Spalt evakuiert oder an einen Kühlkreislauf angeschlossen werden kann. Für eine modifizierten Strömungsweg des Reaktionsmediums entlang der Tauchlampe 1A kann oder können z. B. ein oder mehrere transparente Rohre (nicht dargestellt) um die Tauchlampe gewunden werden. Zum Schutz oder zur Temperierung kann das Innere des Photoreaktors 101 dabei mit entsprechendem gasförmigen oder flüssigen Fluid gefüllt oder gespült werden.

Die in **Fig. 9** skizzierte Vorrichtung 100 zur Durchführung einer photochemischen Reaktion sieht die Anordnung von LED-Flächenstrahlern 1B an den Seitenwänden des Photoreaktors 101 vor, die daher aus einem für die von den LED-Flächenstrahlern 1B strahlungstransparenten Material bestehen oder eine Öffnung aufweisen, in der der LED-Flächenstrahler 1B mit der Fensterscheibe 10a nach innen weisend abgedichtet angeordnet wird.

### BEZUGSZEICHENLISTE

- 1: LED-Lampenmodul
- 2: LED-Gruppe
- 3: LED
- 4: Treibervorrichtung
- 5: Platine
- 6: Tragkäfig
- 6a, 6b, 6c, 6d: Tragstruktur, Stützstruktur, Schienenstruktur, Rohrstruktur
- 7: Versorgungsleitung
- 7a: Anschlussvorrichtung
- 8, 8a: Kühlmittelzulaufabschnitt, Zulauföffnung
- 9: Steuer- und/oder Regelelement
- 9a, 9b, 9c, 9d: Strom-, Spannungs-, Temperatur-, Photonenmessvorrichtung
- 9e: Schalterelement
- 10: Hüllrohr
- 10a: Scheibe
- 11: Innenraum
- 12, 12a, 12b: Gehäuse, Basis, Seitenwand
- 12c, 12d: Zu-, Ablauföffnung
- 13, 14, 14a: Zulauf-, Ablaufanschluss, Ablauföffnung
- 15, 15a: Kopfteil, Durchtrittsöffnung
- 100: Vorrichtung zur Durchführung einer photochemischen Reaktion
- 101: Photoreaktor
- 102: Schaltschrank
- L: Elektrisch nichtleitende Kühlflüssigkeit
- K: Kühlkreislauf
- Iₖₒₙₛₜ: Konstantstromquelle
- Uₖₒₙₛₜ: Konstantspannungsquelle

## Patentansprüche

1. LED-Lampenmodul (1), ausgebildet zur Anordnung in einer Vorrichtung (100) zur Durchführung einer photochemischen Reaktion, wobei das LED-Lampenmodul (1) eine Vielzahl LEDs (3) und einen formgebenden Trägerkörper (6) aufweist, an dem die LEDs (3) befestigt sind, wobei der Trägerkörper (6) mit den LEDs (3) in einem Innenraum (11) des LED-Lampenmoduls (1) angeordnet ist, der von einem transparenten Wandelement (10, 10a) und von einem Gehäuseelement (12, 15) begrenzt wird, wobei das transparente Wandelement (10, 10a) korrespondierend zu den LEDs (3) angeordnet ist, und sich zumindest eine elektrische Versorgungsleitung (7) zum elektrischen Anschluss der LEDs (3) durch das Gehäuseelement (12, 15) erstreckt,
und wobei die Vielzahl LEDs (3) in LED-Gruppen (2) unterteilt ist, die eine vorbestimmte Anzahl LEDs (3) aufweisen, wobei die LEDs (3) jeder LED-Gruppe (2) in Reihe geschaltet sind, und jede LED-Gruppe (2) jeweils einer Treibervorrichtung (4) als Konstantstromquelle (Iₖₒₙₛₜ) zum Betrieb der LEDs (3) der jeweiligen LED-Gruppe (2) zugeordnet und mit dieser verbunden ist,
**dadurch gekennzeichnet, dass**
jede Treibervorrichtung (4) benachbart zu der ihr zugeordneten LED-Gruppe (2) an dem Trägerkörper (6) im Innenraum (11) des LED-Lampenmoduls (1) angeordnet ist, und
jede Treibervorrichtung (4) mit der jeweils zugeordneten LED-Gruppe (2) in Reihe geschaltet ist und einen LED-Stromzweig bildet, wobei die LED-Stromzweige parallel geschaltet sind und die parallel geschalteten LED-Stromzweige über die Versorgungsleitung (7) mit einer Konstantspannungsquelle (Uₖₒₙₛₜ) verbunden sind.

2. LED-Lampenmodul (1) nach Anspruch 1,
**dadurch gekennzeichnet, dass**
die vorbestimmte Anzahl LEDs (3) aller LED-Gruppen (2) gleich ist und höchstens 25, bevorzugt höchstens 20 und besonders bevorzugt höchstens 15 beträgt.

3. LED-Lampenmodul (1) nach Anspruch 1 oder 2,
**dadurch gekennzeichnet, dass**
die LED-Stromzweige aus den LED-Gruppen (2) mit den ihnen zugeordneten Treibervorrichtungen (4) als integrierte Schaltbausteine an Platinen (5) ausgebildet sind, die an dem Trägerkörper (6) befestigt sind, wobei an jeder Platine (5) eine oder mehrere der LED-Gruppen (2) mit der ihnen zugeordneten Treibervorrichtung (4) angeordnet ist/sind.

4. LED-Lampenmodul (1) nach zumindest einem der Ansprüche 1 bis 3,
**dadurch gekennzeichnet, dass**
jeder LED-Gruppe (2) zumindest ein Steuer- und/oder Regelelement (9) und
- zumindest eine Messvorrichtung (9a, 9b, 9c, 9d), die ausgewählt ist aus einer Gruppe umfassend eine Strommessvorrichtung (9a), eine Spannungsmessvorrichtung (9b), eine Temperaturmessvorrichtung (9c) und eine Photonenmessvorrichtung (9d), und/oder
- zumindest ein Schalterelement (9e) zugeordnet ist,
wobei das Steuer- und/oder Regelelement (9) mit der zumindest einen Messvorrichtung (9a, 9b, 9c, 9d) kommunikativ verbunden und dazu ausgebildet ist, in Abhängigkeit eines von der zumindest einen Messvorrichtung (9a, 9b, 9c, 9d) erfassten Messwertes
- das Schalterelement (9e) anzusteuern, um die LEDs (3) der zugeordneten LED-Gruppe (2) in Abhängigkeit der Messwerte an- oder abzuschalten und/oder zu dimmen, und/oder
- die Treibervorrichtung (4) der zugeordneten LED-Gruppe (2) zur Regelung des Konstantstroms (Iₖₒₙₛₜ) anzusteuern, um die LEDs (3) der zugeordneten LED-Gruppe (2) mit einer vorbestimmten Leistung zu betreiben.

5. LED-Lampenmodul (1) nach Anspruch 4,
**dadurch gekennzeichnet, dass**
die Messvorrichtung (9a, 9b, 9c, 9d) und/oder das Schalterelement (9e) als integrierte Schaltbausteine auf der Platine (5) der zugeordneten LED-Gruppe (2) angeordnet sind.

6. LED-Lampenmodul (1) nach Anspruch 4 oder 5,
**dadurch gekennzeichnet, dass**
das Steuer- und/oder Regelelement (9) als integrierter Schaltbaustein auf der Platine (5) der zugeordneten LED-Gruppe (2) oder außerhalb des Innenraums (11) angeordnet ist, wobei die Steuer- und/oder Regelelemente (9) miteinander und/oder mit einer übergeordneten Steuer- und/oder Regeleinheit (90) kommunikativ verbunden und dazu ausgebildet sind, die vorbestimmte Strahlungsleistung der LEDs (3) jeder LED-Gruppe (2) aufeinander abzustimmen, und/oder jeder LED-Gruppe (2) eine ID zuzuweisen, und/oder Betriebsdaten und/oder Betriebszeit jeder LED-Gruppe (2) zu erfassen.

7. LED-Lampenmodul (1) nach zumindest einem der Ansprüche 1 bis 6,
**dadurch gekennzeichnet, dass**
das LED-Lampenmodul (1) zumindest eine Versorgungsschiene (6c) zur Aufnahme der zumindest einen Leitung (7) innerhalb des Innenraums (11) aufweist.

8. LED-Lampenmodul (1) nach zumindest einem der Ansprüche 1 bis 7,
**dadurch gekennzeichnet, dass**
der Trägerkörper (6) ein Tragkäfig (6) mit einer Gitterstruktur ist, der Tragstrukturen (6a), an denen die LED-Gruppen (2) befestigt sind, und zumindest eine Stützstruktur (6b) zur positionierten Anordnung innerhalb des Innenraums (11) aufweist.

9. LED-Lampenmodul (1) nach Anspruch 8,
**dadurch gekennzeichnet, dass**
die zumindest eine Versorgungsschiene (6c) durch zumindest eine Schienenstruktur (6c) des Tragkäfigs (6) bereitgestellt wird.

10. LED-Lampenmodul (1) nach zumindest einem der Ansprüche 1 bis 9,
**dadurch gekennzeichnet, dass**
das LED-Lampenmodul (1) einen Kühlkreislauf (K) mit einer elektrisch nichtleitenden Flüssigkeit (L) aufweist, wobei das Gehäuseelement (12, 15) einen Zulaufanschluss (13) und einen Ablaufanschluss (14) zur Verbindung des Innenraums (11) mit dem Kühlkreislauf (K) aufweist, sodass der Innenraum (11) vollständig mit der elektrisch nichtleitenden Flüssigkeit (L) gefüllt ist.

11. LED-Lampenmodul (1) nach zumindest einem der Ansprüche 1 bis 10,
**dadurch gekennzeichnet, dass**
das LED-Lampenmodul (1) als Tauchlampe (1A) mit einer Längsachse (A) ausgebildet ist, wobei das transparente Wandelement (10, 10a) ein transparentes, einseitig geschlossenes Hüllrohr (10) ist, das koaxial um den Trägerkörper (6) mit den LEDs (3) angeordnet ist, und wobei das Gehäuseelement (12, 15) ein Kopfteil (15) aufweist, das an einem offenen Ende des Hüllrohrs (10) angeordnet ist.

12. LED-Lampenmodul (1) nach Anspruch 11,
**dadurch gekennzeichnet, dass**
der als Tragkäfig (6) ausgebildete Trägerkörper (6) an zumindest einem längsaxialen Ende die zumindest eine Stützstruktur (6b) zur positionierten Anordnung innerhalb des Innenraums (11) aufweist, wobei die Stützstruktur (6b) zur zentrierten Anordnung an dem Kopfteil (15) und/oder dem geschlossenen Ende des Hüllrohrs (10) ausgebildet ist, und/oder
das Kopfteil (15) Durchtrittsöffnungen (15a) aufweist, wobei sich der Zulaufanschluss (13) und der Ablaufanschluss (14) jeweils durch eine der Durchtrittsöffnungen (15a) erstrecken oder damit verbunden sind, und wobei der Zulaufanschluss (13) bevorzugt innenraumseitig mit einer Rohrstruktur (6d) verbunden ist, die in dem als Tragkäfig (6) ausgebildeten Trägerkörper (6) angeordnet ist und sich längs durch den Tragkäfig (6) als Kühlmittelzulaufabschnitt (8) erstreckt, der eine Zulauföffnung (8a) an einem kopfteilfernen Ende des Innenraums (11) bereitstellt, wobei der Ablaufanschluss (14), der sich durch das Kopfteil (15) erstreckt, oder die mit dem Ablaufanschluss (14) verbundene Durchtrittsöffnung (15a) eine Ablauföffnung (14a) an einem kopfteilnahen Ende des Innenraums (11) bereitstellt.

13. LED-Lampenmodul (1) nach zumindest einem der Ansprüche 1 bis 10,
**dadurch gekennzeichnet, dass**
das LED-Lampenmodul (1) als Flächenstrahler (1B) ausgebildet ist, wobei das transparente Wandelement (10, 10a) eine transparente ebene Fensterscheibe (10a) ist, und das Gehäuseelement (12, 15) ein Gehäuse (12) ist, das eine Basis (12a) und Seitenwände (12b) aufweist, wobei die Fensterscheibe (10a) eine von Seitenwände (12b) begrenzte offene Fläche schließt und die an dem Trägerkörper (6) angeordneten LEDs (3) in einer zu der Fensterscheibe (10a) parallelen Ebene (B) zwischen der Fensterscheibe (10a) und der Basis (12a) angeordnet sind.

14. LED-Lampenmodul (1) nach Anspruch 13,
**dadurch gekennzeichnet, dass**
der als Tragkäfig (6) ausgebildete Trägerkörper (6) mit der zumindest einen Stützstruktur (6b) auf der Basis (12a) des Gehäuses (12) angeordnet ist und die Tragstrukturen (6a), an denen die LEDs (3) befestigt sind, auf einer von der zumindest einen Stützstruktur (6b) abgewandten Seite des Tragkäfigs (6) ausgebildet sind, wobei eine Abmessung des Tragkäfigs (6) zwischen den Tragstrukturen (6a) und der zumindest einen Stützstruktur (6b) einen Abstand der LEDs (3) von der Fensterscheibe (10a) bestimmt,
und/oder
das Gehäuse (12) auf gegenüberliegenden Seiten des Innenraums (11) zumindest eine Zulauföffnung (12c), die mit dem Zulaufanschluss (13) verbunden ist, und zumindest eine Ablauföffnung (12d) aufweist, die mit dem Ablaufanschluss (12) verbunden ist.

15. Vorrichtung (100) zur Durchführung einer photochemischen Reaktion, die einen Photoreaktor (101) aufweist, an oder in dem zumindest ein Lampenmodul, das Strahlung mit einer für die photochemische Reaktion geeigneten Wellenlänge emittiert, angeordnet ist,
**dadurch gekennzeichnet, dass**
das Lampenmodul ein LED-Lampenmodul (1) nach zumindest einem der Ansprüche 1 bis 14 ist.

## Claims

1. An LED lamp module (1), formed for the arrangement in a device (100) for carrying out a photochemical reaction, wherein the LED lamp module (1) has a plurality of LEDs (3) and a shaping carrier body (6), to which the LEDs (3) are fastened, wherein the carrier body (6) is arranged with the LEDs (3) in an interior space (11) of the LED lamp module (1), which is delimited by a transparent wall element (10, 10a) and by a housing element (12, 15), wherein the transparent wall element (10, 10a) is arranged in a corresponding manner to the LEDs (3) and at least one electrical supply line (7) for the electrical connection of the LEDs (3) extends through the housing element (12, 15),
and wherein the plurality of LEDs (3) is divided into LED groups (2), which have a predetermined number of LEDs (3), wherein the LEDs (3) of each LED group (2) are connected in series, and each LED group (2) is in each case assigned to a driver device (4) as constant current source (I_{const}) for the operation of the LEDs (3) of the respective LED group (2) and is connected thereto,
**characterized in that**
each driver device (4) is arranged adjacent to the LED group (2) assigned to it on the carrier body (6) in the interior space (11) of the LED lamp module (1), and
each driver device (4) is connected in series to the respective assigned LED group (2) and forms an LED current branch, wherein the LED current branches are connected in parallel and the LED current branches connected in parallel are connected to a constant voltage source (U_{const}) via the supply line (7).

2. The LED lamp module (1) according to claim 1,
**characterized in that**
the predetermined number of LEDs (3) of all LED groups (2) is identical and is maximally 25, preferably maximally 20 and particularly preferably maximally 15.

3. The LED lamp module (1) according to claim 1 or 2,
**characterized in that**
the LED current branches from the LED groups (2) with the driver devices (4) assigned to them are formed as integrated circuit components on printed circuit boards (5), which are fastened to the carrier body (6), wherein one or several of the LED groups (2) with the driver device (4) assigned to them is/are arranged on each printed circuit board (5).

4. The LED lamp module (1) according to at least any one of claims 1 to 3,
**characterized in that**
at least one control and/or regulating element (9) and
- at least one measuring device (9a, 9b, 9c, 9d), which is selected from a group comprising a current measuring device (9a), a voltage measuring device (9b), a temperature measuring device (9c) and a photon measuring device (9d), and/or
- at least one switch element (9e) is assigned to each LED group (2),
wherein the control and/or regulating element (9) is communicatively connected to the at least one measuring device (9a, 9b, 9c, 9d) and is formed as a function of a measuring value captured by the at least one measuring device (9a, 9b, 9c, 9d)
- to control the switch element (9e), in order to activate or deactivate and/or to dim the LEDs (3) of the assigned LED group (2) as a function of the measuring values and/or
- to control the driver device (4) of the assigned LED group (2) for regulating the constant current (I_{const}), in order to operate the LEDs (3) of the assigned LED group (2) with a predetermined power.

5. The LED lamp module (1) according to claim 4,
**characterized in that**
the measuring device (9a, 9b, 9c, 9d) and/or the switch element (9e) are arranged on the printed circuit board (5) of the assigned LED group (2) as integrated circuit components.

6. The LED lamp module (1) according to claim 4 or 5,
**characterized in that**
the control and/or regulating element (9) is arranged on the printed circuit board (5) of the assigned LED group (2) or outside of the interior space (11) as integrated circuit component, wherein the control and/or regulating elements (9) are communicatively connected to one another and/or to a higher-ranking control and/or regulating unit (90) and are formed to adapt the predetermined radiation power of the LEDs (3) of each LED group (2) to one another and/or to assign an ID to each LED group (2) and/or to collect operating data and/or operating time of each LED group (2).

7. The LED lamp module (1) according to at least any one of claims 1 to 6, **characterized in that**
the LED lamp module (1) has at least one supply rail (6c) for receiving the at least one line (7) within the interior space (11).

8. The LED lamp module (1) according to at least any one of claims 1 to 7, **characterized in that**
the carrier body (6) is a carrying cage (6) with a grid structure, of the carrying structures (6a), to which the LED groups (2) are fastened, and has at least one support structure (6b) for the positioned arrangement within the interior space (11).

9. The LED lamp module (1) according to claim 8,
**characterized in that**
the at least one supply rail (6c) is provided by at least one rail structure (6c) of the carrying cage (6).

10. The LED lamp module (1) according to at least any one of claims 1 to 9, **characterized in that**
the LED lamp module (1) has a cooling circuit (K) with an electrically non-conductive liquid (L), wherein the housing element (12, 15) has an inlet connection (13) and a discharge connection (14) for the connection of the interior space (11) to the cooling circuit (K), so that the interior space (11) is completely filled with the electrically non-conductive liquid (L).

11. The LED lamp module (1) according to at least any one of claims 1 to 10, **characterized in that**
the LED lamp module (1) is formed as an immersion lamp (1A) with a longitudinal axis (A), wherein the transparent wall element (10, 10a) is a transparent cladding tube (10), which is closed on one side and which is arranged coaxially around the carrier body (6) with the LEDs (3), and wherein the housing element (12, 15) has a head part (15), which is arranged on an open end of the cladding tube (10).

12. The LED lamp module (1) according to claim 11,
**characterized in that**
the carrier body (6) formed as carrying cage (6) has, on at least one longitudinally axial end, the at least one support structure (6b) for the positioned arrangement within the interior space (11), wherein the support structure (6b) is formed for the centered arrangement on the head part (15) and/or the closed end of the cladding tube (10), and/or
the head part (15) has passage openings (15a), wherein the inlet connection (13) and the discharge connection (14) in each case extend through one of the passage openings (15a) or are connected thereto, and wherein on the side of the interior space, the inlet connection (13) is preferably connected to a tube structure (6d), which is arranged in the carrier body (6) formed as carrying case (6) and which extends longitudinally through the carrying cage (6) as coolant inlet section (8), which provides an inlet opening (8a) on an end of the interior space (11) spaced apart from the head part, wherein the discharge connection (14), which extends through the head part (15), or the passage opening (15a) connected to the discharge connection (14) provides a discharge opening (14a) on an end of the interior space (11) close to the head part.

13. The LED lamp module (1) according to at least any one of claims 1 to 10, **characterized in that**
the LED lamp module (1) is formed as surface radiator (1B), wherein the transparent wall element (10, 10a) is a transparent flat window pane (10a), and the housing element (12, 15) is a housing (12), which has a base (12a) and side walls (12b), wherein the window pane (10a) closes an open surface, which is delimited by side walls (12b) and the LEDs (3) arranged on the carrier body (6) are arranged between the window pane (10a) and the base (12a) in a plane (B) parallel to the window pane (10a).

14. The LED lamp module (1) according to claim 13,
**characterized in that**
the carrier body (6) formed as carrying cage (6) is arranged with the at least one support structure (6b) on the base (12a) of the housing (12), and the carrying structures (6a), to which the LEDs (3) are fastened, are formed on a side of the carrying cage (6) facing away from the at least one support structure (6b), wherein a dimension of the carrying cage (6) between the carrying structures (6a) and the at least one support structure (6b) determines a distance of the LEDs (3) from the window pane (10a),
and/or
the housing (12) has, on opposite sides of the interior space (11), at least one inlet opening (12c), which is connected to the inlet connection (13), and at least one discharge opening (12d), which is connected to the discharge connection (12).

15. A device (100) for carrying out a photochemical reaction, which has a photoreactor (101), on or in which at least one lamp module is arranged, which emits radiation with a wavelength, which is suitable for the photochemical reaction,
**characterized in that**
the lamp module is an LED lamp module (1) according to at least any one of claims 1 to 14.

## Revendications

1. Module de lampe à LED (1), conçu pour être placé dans un dispositif (100) permettant la réalisation d'une réaction photochimique, le module de lampe à LED (1) présentant une pluralité de LED (3) et un corps de support (6) de mise en forme, sur lequel les LED (3) sont fixées, le corps de support (6) avec les LED (3) étant disposé dans un espace intérieur (11) du module de lampe à LED (1), qui est délimité par un élément de paroi transparent (10, 10a) et par un élément de boîtier (12, 15), l'élément de paroi transparent (10, 10a) étant disposé de manière à correspondre aux LED (3), et au moins une ligne d'alimentation électrique (7) pour le raccordement électrique des LED (3) s'étendant par l'élément de boîtier (12, 15),
et la pluralité de LED (3) étant divisée en groupes de LED (2), lesquels comprennent un nombre prédéterminé de LED (3), les LED (3) de chaque groupe de LED (2) étant connectées en série, et chaque groupe de LED (2) étant associé à et connecté à un dispositif pilote (4) respectif en tant que source de courant constant (Iₖₒₙₛₜ) pour le fonctionnement des LED (3) du groupe de LED (2) respectif,
**caractérisé en ce que**
chaque dispositif pilote (4) est disposé de manière adjacente au groupe de LED (2) qui lui est associé sur le corps de support (6) dans l'espace intérieur (11) du module de lampe à LED (1), et
chaque dispositif pilote (4) est monté en série avec le groupe de LED (2) respectivement associé et forme une branche de courant de LED, les branches de courant de LED étant montées en parallèle et les branches de courant de LED montées en parallèle étant reliées à une source de tension constante (Uₖₒₙₛₜ) par la ligne d'alimentation (7).

2. Module de lampe à LED (1) selon la revendication 1,
**caractérisé en ce que**
le nombre prédéterminé de LED (3) de tous les groupes de LED (2) est égal et est au maximum de 25, préférablement au maximum de 20 et plus préférablement au maximum de 15.

3. Module de lampe à LED (1) selon la revendication 1 ou 2,
**caractérisé en ce que**
les branches de courant de LED provenant des groupes de LED (2) avec les dispositifs pilotes (4) qui leur sont associés sont conçues sous forme de modules de commutation intégrés sur des platines (5) qui sont fixées sur le corps de support (6), un ou plusieurs des groupes de LED (2) avec le dispositif pilote (4) qui leur est associé est/étant disposé(s) sur chaque platine (5).

4. Module de lampe à LED (1) selon au moins l'une des revendications 1 à 3, **caractérisé en ce que**
à chaque groupe de LED (2) sont associés au moins un élément de commande et/ou de régulation (9) et
- au moins un dispositif de mesure (9a, 9b, 9c, 9d) sélectionné dans un groupe comprenant un dispositif de mesure de courant (9a), un dispositif de mesure de tension (9b), un dispositif de mesure de température (9c) et un dispositif de mesure de photons (9d), et/ou
- au moins un élément de commutation (9e),
l'élément de commande et/ou de régulation (9) étant relié par communication à l'au moins un dispositif de mesure (9a, 9b, 9c, 9d) et étant conçu pour, en fonction d'une valeur de mesure détectée par l'au moins un dispositif de mesure (9a, 9b, 9c, 9d),
- commander l'élément de commutation (9e) pour, en fonction des valeurs de mesure, allumer ou éteindre et/ou faire varier la luminosité des LED (3) du groupe de LED (2) associé, et/ou
- commander le dispositif pilote (4) du groupe de LED (2) associé pour la régulation du courant constant (Iₖₒₙₛₜ), afin de faire fonctionner les LED (3) du groupe de LED (2) associé avec une puissance prédéterminée.

5. Module de lampe à LED (1) selon la revendication 4,
**caractérisé en ce que**
le dispositif de mesure (9a, 9b, 9c, 9d) et/ou l'élément de commutation (9e) sont disposés en tant que modules de commutation intégrés sur la platine (5) du groupe de LED (2) associé.

6. Module de lampe à LED (1) selon la revendication 4 ou 5,
**caractérisé en ce que**
l'élément de commande et/ou de régulation (9) est disposé en tant que module de commutation intégré sur la platine (5) du groupe de LED (2) associé ou à l'extérieur de l'espace intérieur (11), les éléments de commande et/ou de régulation (9) étant reliés par communication entre eux et/ou à une unité de commande et/ou de régulation (90) supérieure et étant conçus pour harmoniser entre elles les puissances de rayonnement prédéterminées des LED (3) de chaque groupe de LED (2), et/ou pour attribuer un ID à chaque groupe de LED (2), et/ou saisir les données de fonctionnement et/ou le temps de fonctionnement de chaque groupe de LED (2).

7. Module de lampe à LED (1) selon au moins l'une des revendications 1 à 6, **caractérisé en ce que**
le module de lampe à LED (1) présente au moins un rail d'alimentation (6c) pour recevoir l'au moins un câble (7) à l'intérieur de l'espace intérieur (11).

8. Module de lampe à LED (1) selon au moins l'une des revendications 1 à 7, **caractérisé en ce que**
le corps de support (6) est une cage de support (6) avec une structure en treillis, qui présente des structures de support (6a) auxquelles les groupes de LED (2) sont fixés, et au moins une structure d'appui (6b) pour une disposition positionnée à l'intérieur de l'espace intérieur (11).

9. Module de lampe à LED (1) selon la revendication 8,
**caractérisé en ce que**
l'au moins un rail d'alimentation (6c) est fourni par au moins une structure de rail (6c) de la cage de support (6).

10. Module de lampe à LED (1) selon au moins l'une des revendications 1 à 9, **caractérisé en ce que**
le module de lampe à LED (1) présente un circuit de refroidissement (K) avec un liquide (L) électriquement non conducteur, l'élément de boîtier (12, 15) présentant un raccord d'entrée (13) et un raccord de sortie (14) pour relier l'espace intérieur (11) au circuit de refroidissement (K), de façon à ce que l'espace intérieur (11) soit entièrement rempli du liquide (L) électriquement non conducteur.

11. Module de lampe à LED (1) selon au moins l'une des revendications 1 à 10, **caractérisé en ce que**
le module de lampe à LED (1) est conçu sous forme de lampe à immersion (1A) avec un axe longitudinal (A), l'élément de paroi transparent (10, 10a) étant un tube de gainage (10) transparent, fermé d'un côté, qui est disposé coaxialement autour du corps de support (6) avec les LED (3), et l'élément de boîtier (12, 15) présentant une partie supérieure (15) qui est disposée à une extrémité ouverte du tube de gainage (10).

12. Module de lampe à LED (1) selon la revendication 11,
**caractérisé en ce que**
le corps de support (6) conçu sous forme de cage de support (6) présente, à au moins une extrémité longitudinale, l'au moins une structure d'appui (6b) pour une disposition positionnée à l'intérieur de l'espace intérieur (11), la structure d'appui (6b) étant conçue pour une disposition centrée sur la partie supérieure (15) et/ou l'extrémité fermée du tube de gainage (10), et/ou
la partie supérieure (15) présente des ouvertures de passage (15a), le raccord d'entrée (13) et le raccord de sortie (14) s'étendant chacun par l'une des ouvertures de passage (15a) ou étant reliés à celles-ci, et le raccord d'entrée (13) étant relié, de préférence du côté de l'espace intérieur, à une structure tubulaire (6d) qui est disposée dans le corps de support (6) conçu sous forme de cage de support (6) et qui s'étend longitudinalement par la cage de support (6) en tant que section d'entrée de réfrigérant (8), qui fournit une ouverture d'entrée (8a) à une extrémité de l'espace intérieur (11) éloignée de la partie supérieure, le raccord de sortie (14) qui s'étend par la partie supérieure (15) ou l'ouverture de passage (15a) reliée au raccord de sortie (14) fournissant une ouverture de sortie (14a) à une extrémité de l'espace intérieur (11) proche de la partie supérieure.

13. Module de lampe à LED (1) selon au moins l'une des revendications 1 à 10, **caractérisé en ce que**
le module de lampe à LED (1) est conçu comme projecteur de surface (1B), l'élément de paroi transparent (10, 10a) étant une vitre de fenêtre plane transparente (10a), et l'élément de boîtier (12, 15) étant un boîtier (12) qui présente une base (12a) et des parois latérales (12b), la vitre de fenêtre (10a) fermant une surface ouverte délimitée par des parois latérales (12b) et les LED (3) disposées sur le corps de support (6) étant disposées dans un plan (B) parallèle à la vitre de fenêtre (10a), entre la vitre de fenêtre (10a) et la base (12a).

14. Module de lampe à LED (1) selon la revendication 13,
**caractérisé en ce que**
le corps de support (6) conçu sous forme de cage de support (6) est disposé avec l'au moins une structure d'appui (6b) sur la base (12a) du boîtier (12) et les structures de support (6a), sur lesquelles les LED (3) sont fixées, sont formées sur un côté de la cage de support (6) opposé à l'au moins une structure d'appui (6b), une dimension de la cage de support (6) entre les structures de support (6a) et l'au moins une structure d'appui (6b) déterminant une distance des LED (3) par rapport à la vitre de fenêtre (10a),
et/ou
le boîtier (12) présente, sur des côtés opposés de l'espace intérieur (11), au moins une ouverture d'entrée (12c) qui est reliée au raccord d'entrée (13) et au moins une ouverture de sortie (12d) qui est reliée au raccord de sortie (12).

15. Dispositif (100) permettant la réalisation d'une réaction photochimique, qui présente un photoréacteur (101) sur ou dans lequel est disposé au moins un module de lampe qui émet un rayonnement avec une longueur d'onde appropriée pour la réaction photochimique,
**caractérisé en ce que**
le module de lampe est un module de lampe à LED (1) selon au moins l'une des revendications 1 à 14.
